# EUROPEAN PATENT APPLICATION

(11) **EP 4 674 284 A2**
(43) Date of publication of application: **07.01.2026**
(21) Application number: 25204410.2
(22) Date of filing: 29.03.2024
(51) Int. Cl.: A23L 33/00

(54) **A SHELF-STABLE PACKAGED BEVERAGE**

(30) Priority: 31.03.2023 GB 202304892
(62) Divisional of application: 24716369.4
(71) Applicant: Anabio Technologies Limited, Dublin, D02 RW27 (IE)
(72) Inventor: Bleiel, Sinead, Dublin, D02 RW27 (IE)
(74) Representative: Purdylucey Intellectual Property

(57) **Abstract**

A packaged liquid product comprises a container and heat-treated liquid product within the container. The liquid product comprises an aqueous phase and a solid phase comprising dry spray englobed microparticles comprising (a) a core comprising an agglomerate of sub-microparticles comprising live probiotic bacteria and a carrier material, and (b) a first coating of polymerised denatured food grade protein encapsulating the core. The first coating is made in a bottom coating fluidised bed process, that provides excellent protection to the probiotics when a beverage containing the microparticles is subjected to as high heat treatment such as UHT. The heat-treated liquid product is shelf stable for at least 6 months, and least 70% of the live probiotic bacteria in the packaged liquid product remain viable after storage of the packaged liquid product for at least 6 months 25°C.

## Description

### Field of the Invention

The present invention relates to a shelf-stable packaged beverage. The invention also relates to method of making a shelf-stable packaged beverage.

### Background to the Invention

Apart from acidic fermentation, most shelf-stable beverages have to be heat-treated to prevent the beverage spoiling during storage. Ultra Heat Treatment (UHT) is often employed to heat treat such beverages, where the high heat treatment is effective to kill pathogens and spores in the beverage allowing the beverage to be stored for an extended time at ambient temperature. For some beverages, for example beverages that contain heat sensitive components such as vitamins or live probiotic bacteria, high heat treatment methods such as UHT are not ideal as the heat results in destruction or inactivation of active agents and, in the case of live probiotics, a severe reduction in the number of viable probiotics that survive the treatment. For many probiotic beverages, this problem is avoided by using acidic fermented drinks as the vehicle for the probiotic, e.g. kombuchas, although these drinks generally have to be refrigerated and have a limited shelf life. In addition, spore-forming bacteria must be used and the fermented products need to maintain pH levels below pH5 to avoid germination of the cells. Limited health claims are available for spore-forming bacteria such as bacillus; and the use of *bacillus* spored in refrigerated beverages requires significant overdose. Despite overdose levels in excess of 200%, stability of *bacillus* spores remains limited and data shows survival less than 20% of the recommended label claim in acidic refrigerated fermented products.

WO2008/056344 describes the encapsulation of probiotic bacteria in a denatured whey protein matrix using a cold gelation process in which the probiotic bacteria are mixed with a suspension of denatured whey, extruded into microdroplets, which are cured in an acidic bath at a pH of 4.6. The use of microbead technology has the potential to overcome bacteria stability issues in beverages; however the density and particle size of microbeads presents a significant limitation for the beverage sector, due to lack of homogeneity of microbeads in beverages of varied viscosity i.e. dose consistency represent a limiting factor for the use of microbeads in commercial beverage systems. Furthermore, the encapsulation system is dairy based which represent an allergen and an animal food source; thus limiting it's use in a broad range of applications suitable for vegan and vegetarians. In addition, the probiotic concentration / cell load in microbeads is limited to 10 - 20 Billion cells per gram; leading to the use of a larger dose / fortification level in finished products. This results in a cost limitation for the industry since cell load in region of 100 Billion cells per gram represents a tangible and practical dosage level for industrial use of probiotics in beverages.

It is an objective of the invention to overcome at least one of the above-referenced problems. It is a particular object of the invention to provide a packaged shelf-stable beverage containing heat-sensitive active agents (such as live probiotic bacteria) that can be UHT treated without damaging the active agent and can be stored for extended periods of time at ambient temperature without spoilage.

### Summary of the Invention

The objective is met by the provision of a shelf-stable, heat treated (e.g. pasteurised or UHT treated) beverage packaged in a container. The beverage contains spray englobed microparticles with a heat-sensitive active agent such as live probiotic bacteria encapsulated within the microparticles. The spray englobed microparticles comprise a core comprising an agglomeration of sub-microparticles containing the active agent (e.g. live probiotics), a carrier material and optionally a binder, and a coating of polymerised denatured protein surrounding the core. The microparticles are produced by a fluidised bed process (that typically employs a top sprayer) that produces the core and bottom spraying the coating (e.g. using a fluidised bed with a Wurster insert) that coats the core with denatured protein, producing the microparticles. The Applicant has surprisingly found that live probiotics, including non-spore-forming probiotics such as *Lactobacillus* and *Bifidobacterium,* contained within microparticles produced with such a fluidised bed system exhibit a very high survival rate after UHT treatment, allowing long life, shelf-stable, probiotic containing beverages to be produced that contain high levels (e.g. at least 10⁶ CFU/ml) of viable culturable probiotic bacteria after 24 months of storage at ambient temperatures. In addition, the quality and physicochemical properties of the beverage (e.g. pH, viscosity, colour) remain largely unaffected after prolonged storage at ambient temperature storage, which demonstrates the stability of the microparticles to leakage of probiotics into the beverage.

In one aspect, there is provided a composition of dry spray englobed microparticles comprising:
(a) a core comprising an agglomerate of sub-microparticles comprising live probiotic bacteria and a carrier material; and
(b) a first coating of polymerised denatured food grade protein encapsulating the core.

In another aspect, there is described a shelf-stable packaged beverage comprising a container and heat-treated beverage within the container, in which the beverage comprises an aqueous phase and a solid phase comprising dry spray englobed microparticles, in which the dry spray englobed microparticles comprise:
(a) a core comprising an agglomerate of sub-microparticles comprising live probiotic bacteria and a carrier material; and
(b) a first coating of polymerised denatured food grade protein encapsulating the core.

Typically, at least 70% of the live probiotic bacteria in the shelf-stable packaged beverage remain viable after storage of the shelf-stable packaged beverage for at least 6 months at 21°C.

In any embodiment, the agglomerate of sub-microparticles comprising live probiotic bacteria and a carrier material comprises a binding agent selected from lipid and denatured food grade protein.

In any embodiment, the first coating of polymerised denatured food grade protein is applied to the agglomerate of sub-microparticles by bottom spraying the denatured food grade protein on a fluidised bed.

In any embodiment, the heat-treated beverage is a UHT treated beverage.

In any embodiment, the carrier material is selected from native food grade protein and a carbohydrate, or a combination thereof.

In any embodiment, the carrier material is a maltodextrin, optionally in combination with a native food grade protein.

In any embodiment, the dry spray englobed microparticle comprises a second coating comprising a second coating material selected from denatured food grade polymerised protein, lipid and sugar.

In any embodiment, the dry spray englobed microparticle comprises a third coating comprising a third coating material selected from food grade denatured polymerised protein, lipid and sugar.

In any embodiment, the dry spray englobed microparticles consist essentially of probiotic bacteria; native protein and/or carbohydrate; denatured protein and/or lipid; and water.

In any embodiment, in which the dry spray englobed microparticles consist essentially of probiotic bacteria; native protein; carbohydrate; denatured protein; lipid; and water.

In any embodiment, in which the dry spray englobed microparticles consist essentially of probiotic bacteria; maltodextrin; denatured protein; lipid; optionally native protein; and water.

In any embodiment, the heat-treated beverage comprises 10⁴ to 10¹⁰ CFU (colony forming units) of live probiotic bacteria encapsulated within the dry spray englobed microparticles. In any embodiment, the heat-treated beverage comprises 10⁶ to 10⁸ CFU of live probiotic bacteria encapsulated within the dry spray englobed microparticles. In any embodiment, the heat-treated beverage comprises at least 10⁶, 10⁷, 10⁸ or 10⁹ CFU/ml of live probiotic bacteria encapsulated within the dry spray englobed microparticles. In the products of the invention a wide range of probiotics from *Lactobacillus* or *Bifidobacteria* can be included, generally sufficient to consume from 10⁶ to 10⁹ CFU live probiotics per day. Thus, a beverage of the invention can comprise 10⁵ to 10⁶, 10⁶ to 10⁷, 10⁷ to 10⁸, 10⁸ to 10⁹, 10⁹ to 10¹⁰ CFU of probiotics. The beverage of the invention may also comprise 10⁵ to 10⁶, 10⁶ to 10⁷, 10⁷ to 10⁸, 10⁸ to 10⁹, 10⁹ to 10¹⁰ CFU of probiotics per millilitre of beverage. This range may be consumed throughout the day (e.g. 4 times a dose of 10⁸ probiotics to reach a total doses of 4X10⁸ CFU a day, or a beverage of min 80 0 ml that contains 10⁶ CFU/ml which is consumed throughout the day to provide a daily dose of probiotic) to reach a total doses 10⁸ CFU a day but it is also possible to consume one dose a day in a beverage or food product. The number of probiotic units per quantity of the product will depend on the size of a daily portion and will be quantified by traditional plate count or flow cytometry. The shelf-stable, heat treated (e.g. pasteurised or UHT treated) beverage packaged in a container may comprise 50-1000 ml, 50-500 ml, 100-500ml. 100-400 ml, 100-300 ml or 100-200 ml of beverage. The term "CFU" applies to enumeration of probiotic bacteria by plate count. When probiotic bacteria are enumerated by flow cytometry and FACS (as described below), the term CFU as employed above (and herein) may be replaced with AFU (Active Fluorescent Units).

In any embodiment, the coating comprises denatured polymerised plant protein.

In any embodiment, the beverage comprises at least 70%, 80%, 90%, or 95% water (w/w).

In any embodiment, the beverage is selected from a fruit juice, a vegetable juice, a smoothie, a water product, a carbonated beverage, a low acid beverage, a high acid beverage, a protein beverage, a non-dairy beverage, a dairy beverage, a fermented beverage, a non-fermented beverage, a liquid concentrate, or mixtures thereof.

In any embodiment, the probiotic is a non-spore forming bacteria. Examples include probiotic strains of *Lactobacillus* and *Bifidobacterium.*

In any embodiment the dry spray englobed microparticle comprises a prebiotic. In any embodiment, the prebiotic is disposed in the core of the dry spray englobed microparticle. In any embodiment, the prebiotic constitutes about 0.01 % to 2.5 % of the microparticles by weight.

In any embodiment, the dry spray englobed microparticles comprises on a dry weight basis 15-85 % live probiotic material and 15-85 % carrier material (w/w).

In any embodiment, the dry spray englobed microparticles have a water activity (Aw) of less than 0.2.

In any embodiment, the dry spray englobed microparticles have a dimension (Dv90) of about 150 to 500 µm.

In any embodiment, the container is selected from a carton, a bottle (glass or polymer), a can, bag or a tube.

In any embodiment, the container is aseptically filled with the beverage.

In any embodiment, the container is hermetically sealed. In any embodiment, the container is aseptically sealed.

In any embodiment, one or more physicochemical properties of the beverage remain unaffected after 6 months (e.g. 6 to 12 months) storage at 21°C.

In any embodiment, the pH, viscosity and colour of the beverage remain unaffected after 6 months (e.g. 6 to 12 months) storage at 21°C.

In any embodiment, the beverage exhibits no evidence of flocculation or separation after 6 months (e.g. 6 to 12 months) storage at 21°C.

In another aspect, there is provided a method of producing a composition of dry spray englobed microparticles in a process comprising:
fluidising an active agent, for example live probiotic, and a carrier material on a bed of a fluidised bed dryer to produce agglomerates of sub-microparticles comprising live probiotic and carrier material;
coating the agglomerates of sub-microparticles by bottom-spraying denatured food grade protein on to the agglomerates on the fluidised bed to form spray englobed microparticles; and
drying the spray englobed microparticles on the fluidised bed.

In another aspect, there is provided a method of producing a shelf-stable packaged beverage comprising the steps of:
providing dry spray englobed microparticles comprising (a) a core comprising an agglomerate of sub-microparticles comprising live probiotic bacteria and a carrier material; and (b) a first coating of polymerised denatured food grade protein encapsulating the core;
combining the dry spray englobed microparticles with a beverage;
heat treating the beverage containing the dry spray englobed microparticles; and
packaging the beverage in a container to provide the packaged liquid product;
wherein the step of heat treating the beverage is carried out prior to or after the packaging step.

In any embodiment, the heat treatment is UHT treatment or pasteurisation.

In any embodiment, the method comprises heat treating the beverage prior to the packaging, and the packaging step comprises aseptically filling the container with the heat-treated beverage and optionally aseptically sealing the container after it has been filled.

In any embodiment, the method comprises forming the dry spray englobed microparticles in a process comprising:
fluidising live probiotic and a carrier material on a bed of a fluidised bed dryer to produce agglomerates of sub-microparticles comprising live probiotic and carrier material;
coating the agglomerates of sub-microparticles by bottom-spraying denatured food grade protein on to the agglomerates on the fluidised bed to form spray englobed microparticles; and
drying the spray englobed microparticles on the fluidised bed.

In any embodiment, the coating step comprises a step of top-spraying food grade protein on to the agglomerates prior to the bottom-spraying step.

In any embodiment, the coating step comprises bottom spraying a solution of a polymerisation agent on to the coating of denatured food grade protein.

In any embodiment, the polymerisation agent is selected from divalent metal salt such as calcium chloride or an acid such as citric acid.

In any embodiment, the coating step comprises a drying step to reduce the moisture content of the microparticles before the polymerisation agent is bottom sprayed on to the denatured food grade protein coat. Typically, the drying step comprises reducing the moisture content of the microparticles by 25-35%.

In any embodiment, the polymerisation agent is bottom sprayed on to the coating of denatured food grade protein while the coating is still wet.

In any embodiment, the process comprises a plurality of coating steps, in which each step comprises coating the agglomerates with denatured food grade protein, optionally drying the microparticles, and then spraying polymerisation agent on to the coating.

In any embodiment, the process comprises a drying step to reduce the moisture content of the microparticles in between each coating step.

In any embodiment, the fluidisation step comprises fluidising live probiotic, a carrier material and a binder on a bed of a fluidised bed dryer, in which the binder is generally selected from lipid and denatured protein.

In any embodiment, the agglomerates of sub-microparticles are produced in a top-spraying fluidising process.

In any embodiment, the fluidising step comprises adding carrier material to the bed of the fluidised bed, fluidising the carrier material, adding live probiotic to the bed of the fluidised bed, and fluidising the carrier material and live probiotic on the bed to form sub-microparticles, and adding a binder (generally by top-spraying) to the bed of the fluidised bed, and fluidising the binder and sub-microparticles to form agglomerates.

In any embodiment, the binder is sprayed on to the fluidised bed. The binder may be a suspension of denatured protein, optionally comprising oil.

In any embodiment, the method comprises drying the spray englobed microparticles to a water activity (Aw) of less than 0.2.

In any embodiment, the binder comprises denatured food grade protein, and the method comprises a step of top-spraying a polymerising salt onto the agglomerates prior to the coating step.

In another aspect, there is described a packaged beverage (typically shelf stable packaged beverage) produced according to the method of the invention.

In another aspect, there is described a composition comprising or consisting of dry spray englobed microparticles produced according to the method of the invention.

The process for producing the microparticles of the invention employs a fluidised bed (typically a vibrational fluidised bed) to make the agglomerates and a fluidised bed coating process to coat the agglomerates. One embodiment of the process for making the agglomerates generally comprises two stages, a first stage where the agglomerates are made and a second stage where the agglomerates are coated. In the first stage, a carrier material is added to the fluidised bed and fluidised. Then, the active agent (e.g. probiotic material) is added and the bed is further fluidised. A binder (e.g. lipid, or denatured protein) is then generally top-sprayed on to the bed to form the agglomerates while the bed of material is being fluidised.

Once the agglomerates have been made, they are coated in a bottom spray coating fluidised bed process. This process generally employs a Wurster insert on the fluidised bed that sprays coating material and fluidising gas from underneath the agglomerates. In some embodiments, a polymerisation salt is then bottom sprayed on to the microparticles to promote polymerisation of the denatured protein coat. This has been found to produce coatings that provide excellent protection to the active agents in the agglomerates, allowing probiotic-containing microparticles of the invention to be incorporated into beverages which are subsequently UHT treated. Heretofore, this would not have been possible as a high heat treatment (such as UHT) would destroy the live probiotics. The process may employ a concentric nozzle having an inner nozzle and a concentric nozzle in which coating material is sprayed through the inner nozzle and the fluidising gas is pumped through the outer nozzle, or vice versa. The air flow rates are generally in the range of 110 to 300 m³/hr. The air inlet temperature is generally in the region of 35 to 45°C, and the pressure of the bottom spray nozzle is generally about 2 to 4.5 Bar.

The following non-limiting embodiments of spray englobed microparticles are described herein:

### Microparticle 1

Core: Agglomerate of microparticles. Microparticles comprise live probiotic and maltodextrin carrier.

Coating of polymerised denatured plant protein applied by Wurster fluidised bed.

### Microparticle 2

Core: Agglomerate of microparticles. Microparticles comprise live probiotic and native plant protein carrier.

Coating of polymerised denatured plant protein applied by Wurster fluidised bed.

### Microparticle 3

Core: Agglomerate of microparticles. Microparticles comprise live probiotic and maltodextrin/native plant protein carrier. Agglomerate comprises lipid binder.

Coating of polymerised denatured plant protein applied by Wurster fluidised bed.

### Microparticle 4

Core: Agglomerate of microparticles. Microparticles comprise live probiotic and maltodextrin/native plant protein carrier. Agglomerate comprises denatured plant protein binder.

Coating of polymerised denatured plant protein applied by Wurster fluidised bed.

### Microparticle 5

Core: Agglomerate of microparticles. Microparticles comprise live probiotic and maltodextrin/native plant protein carrier.

Inner coating of polymerised denatured plant protein

Outer coating of non-polymerised dried denatured plant protein

Intermediate coating of oil (all coatings applied by Wurster fluidised bed).

### Microparticle 6

Core: Agglomerate of microparticles. Microparticles comprise heat-sensitive micronutrient and maltodextrin/native plant protein carrier.

Inner coating of polymerised denatured plant protein

Outer coating of non-polymerised dried denatured plant protein

Intermediate coating of oil (all coatings applied by Wurster fluidised bed).

### Microparticle 7

Core of active agent (e.g., probiotic) and carrier material

Inner coating of polymerised denatured plant protein

Outer coating of polymerised denatured plant protein

First intermediate coating of oil.

Second intermediate coating of non-polymerised dried denatured plant protein

Other aspects and preferred embodiments of the invention are defined and described in the other claims set out below.

### Brief Description of the Figures

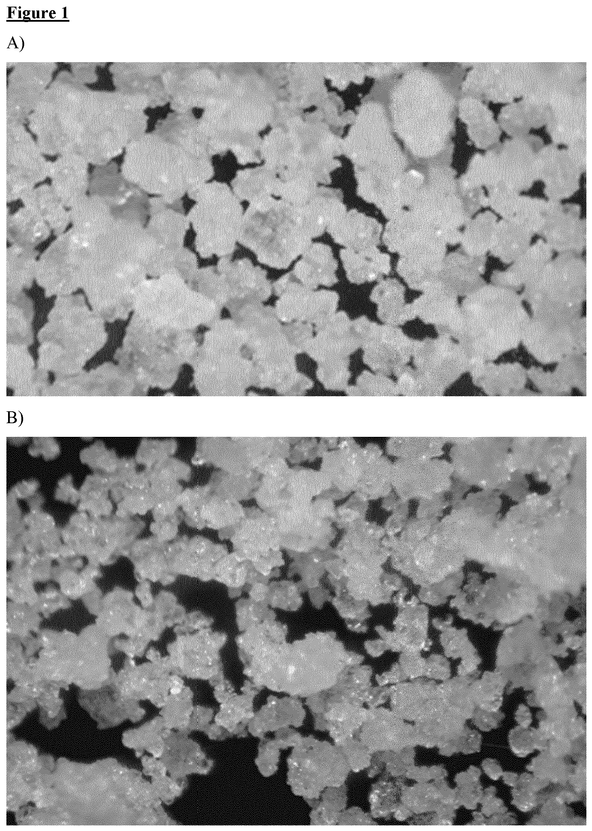

**Figure 1A and 1B** **are** illustrations of agglomerates of sub-microparticles forming part of the spray englobed microparticle of the invention. Core is an agglomerate of microparticles comprising live lactobacillus with a maltodextrin carrier. Light Microscope × 20 magnification.

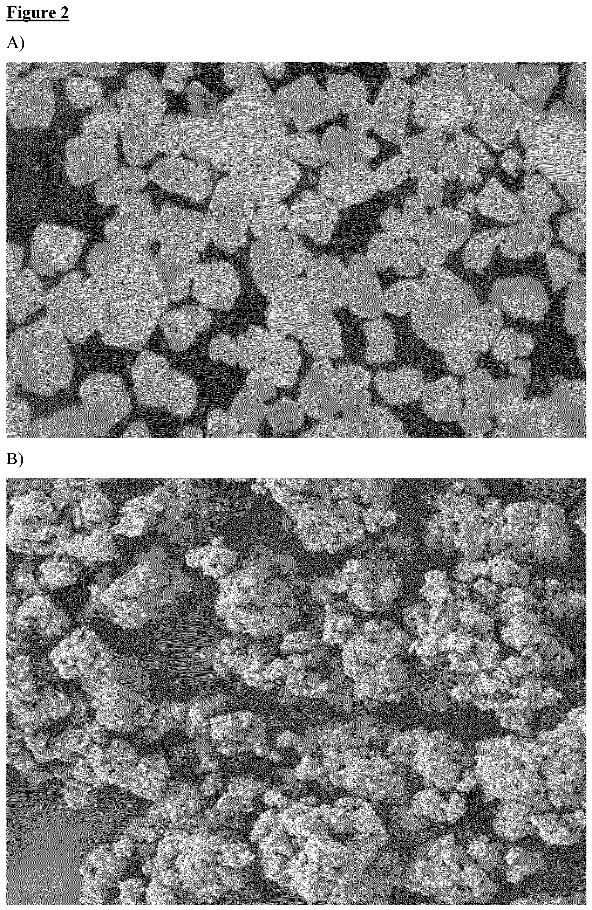

**Figure 2A and 2B** are illustrations of spray englobed microparticles of the invention comprising the agglomerates of Figure 1 englobed within a polymerised denatured plant protein coating. Core is an agglomerate of microparticles comprising live *lactobacillus* and a maltodextrin carrier. Englobed coating consists of a polymerised denatured plant protein applied by Wurster fluidised bed. Figure 2A shows Light Microscope (× 20 magnification) and Figure 2B illustrates a Scanning Electron Microscope with 10 µm range.

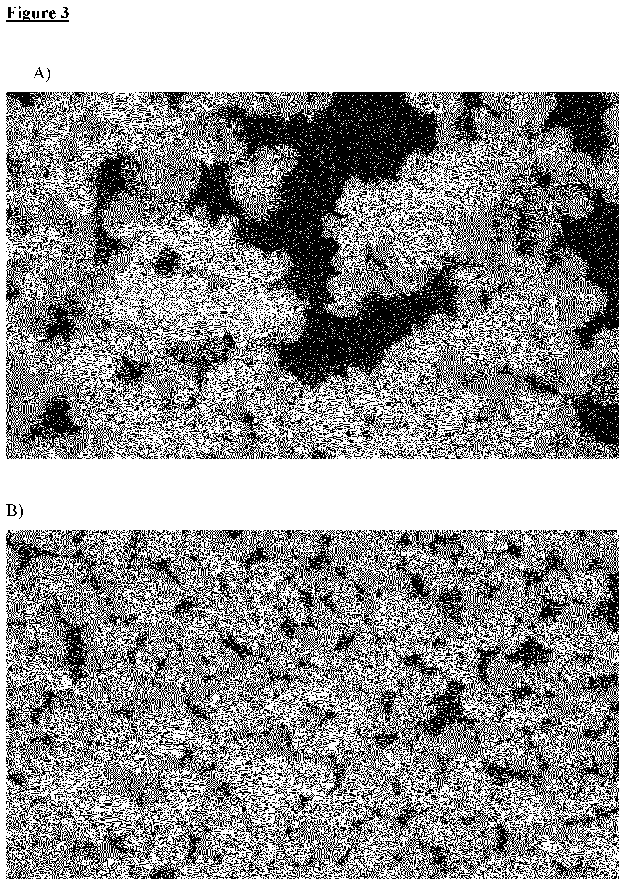

**Figures 3A and 3B** are illustrations of alternative agglomerates of sub-microparticles forming part of the spray englobed microparticle of the invention. Image shows a core of agglomerated microparticles comprising live bifidobacteria and native plant protein carrier. The agglomerate comprises lipid binder. Light Microscope with x5 magnification (A) and × 20 magnification (B).

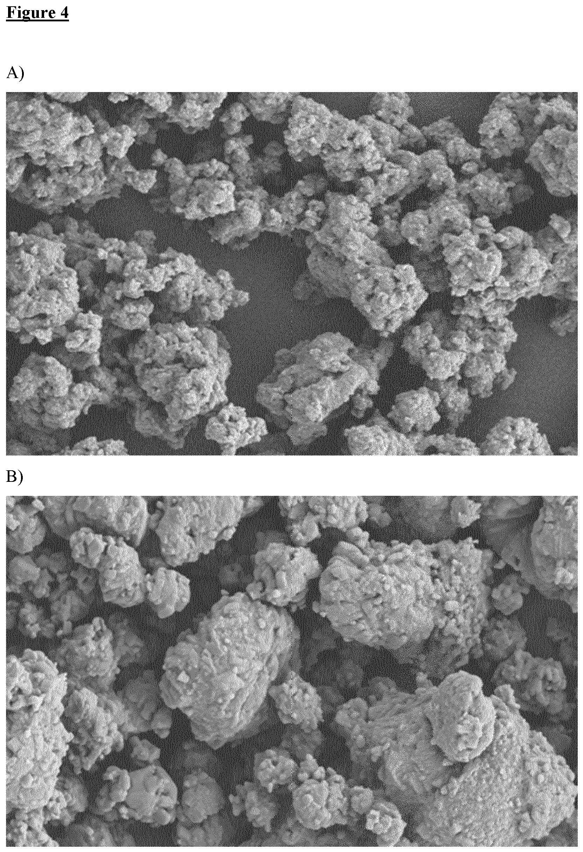

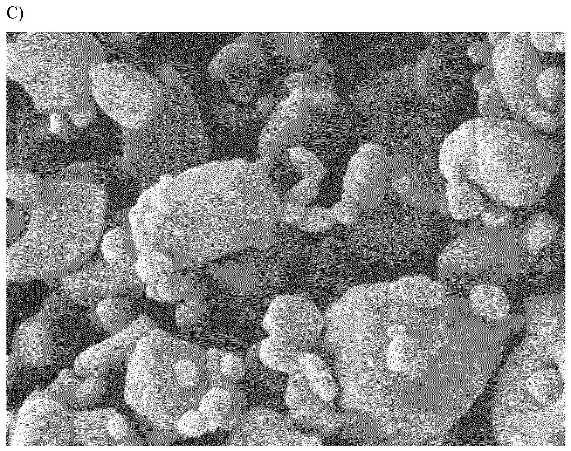

**Figure 4A, 4B** **and** **4C** are illustrations of alternative spray englobed microparticles of the invention showing the agglomerates of Figure 3 englobed within a polymerised denatured plant protein coating. The coating of polymerised denatured plant protein was applied by Wurster fluidised bed.

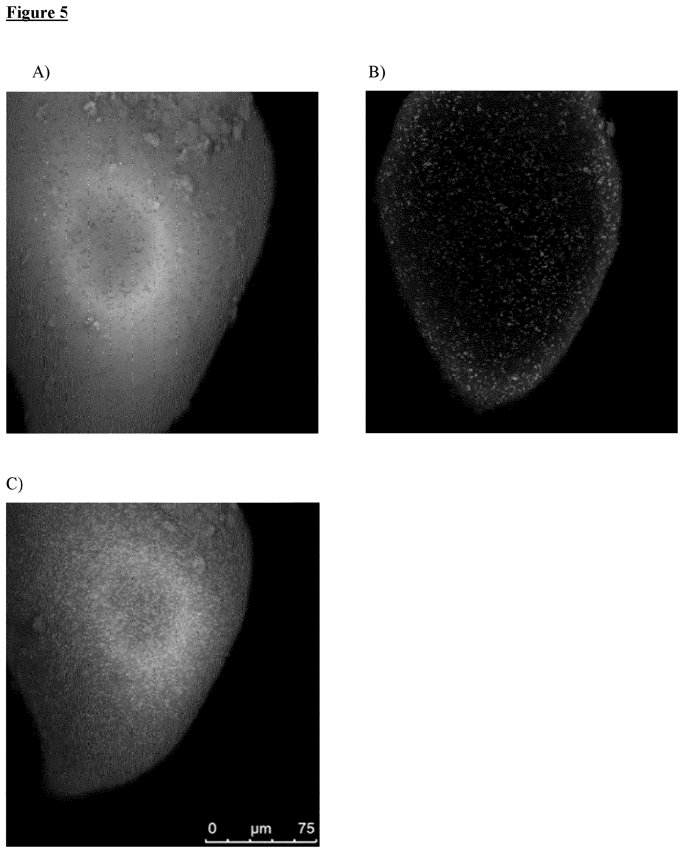

**Figure 5** is an illustration of an alternative spray englobed microparticle of the invention having a first polymerised denatured plant protein coating, a second non-polymerised (dried) denatured plant protein coating, and an intermediate coating of oil. **Figure 5A** relates to a core of agglomerated microparticles comprising probiotic bacteria and a maltodextrin carrier. **Figure 5B** relates to a core of agglomerated microparticles comprising heat-sensitive micronutrient i.e. melatonin and a maltodextrin carrier. The materials consist of an inner coating of polymerised denatured plant protein and an outer coating of non-polymerised dried denatured plant protein with an intermediate coating of oil (all coatings applied by Wurster fluidised bed). Magnification is 75 µm for the surface range and contract lighting enhances the identification of englobed active and surface crevices. **Figure 5C** is a block diagram illustrating another embodiment of the process of forming spray englobed microparticles of the invention after storage stability at elevated temperatures (37°C) using lactobacillus probiotics. This product consists of a probiotic core and maltodextrin carrier; an inner coating of polymerised denatured plant protein; an outer coating of polymerised denatured plant protein and an initial intermediate coating of oil. A second intermediate coating of non-polymerised dried denatured plant protein was allowed applied.

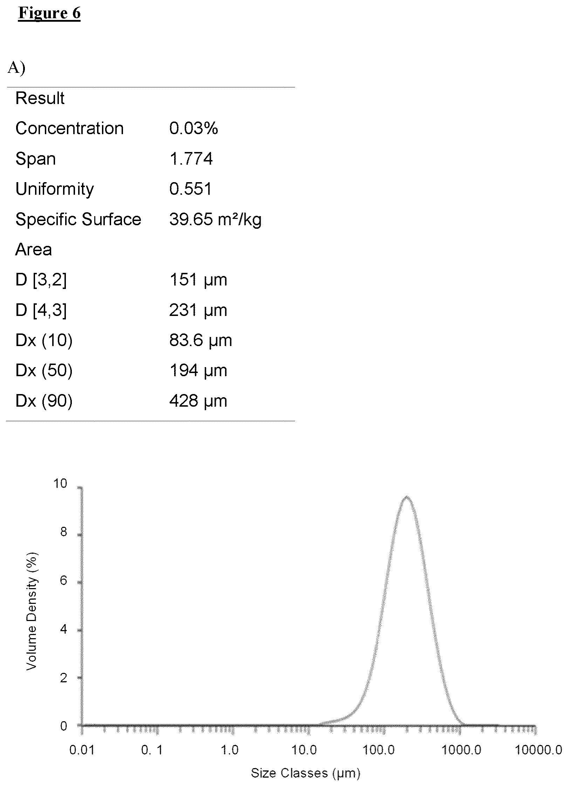

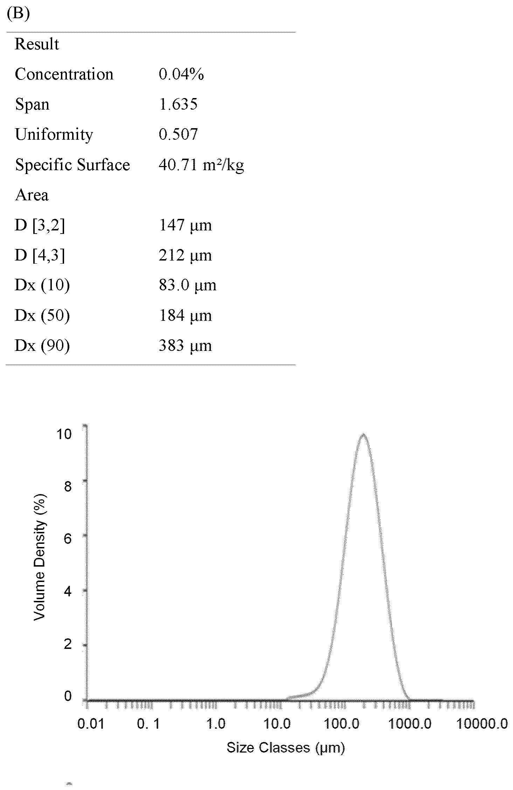

**Figure 6** is a graph illustrating the particle size distribution of spray englobed microparticle with encapsulated (A) *Lactobacillus* and (B) *Bifidobacteria.*

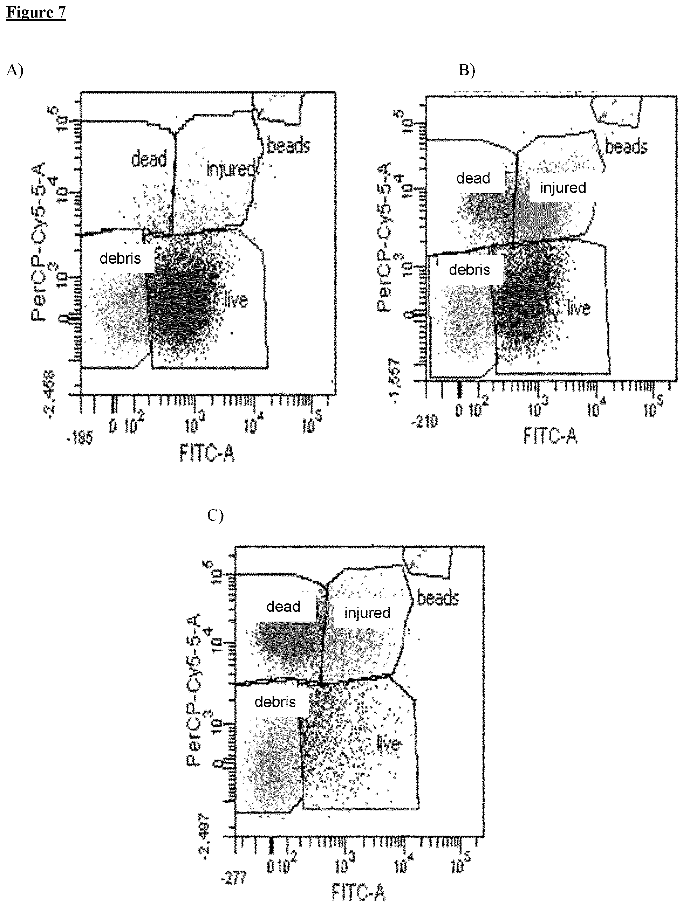

**Figure 7****:** Flow Cytometry dotplots illustrating live cells present in raw culture lyophilised powder (A). Live: dead cell proportions after exposure to beverage pre heat temperatures of 50°C (B). Live: dead cell proportions after exposure to beverage pre heat temperatures of 70°C.

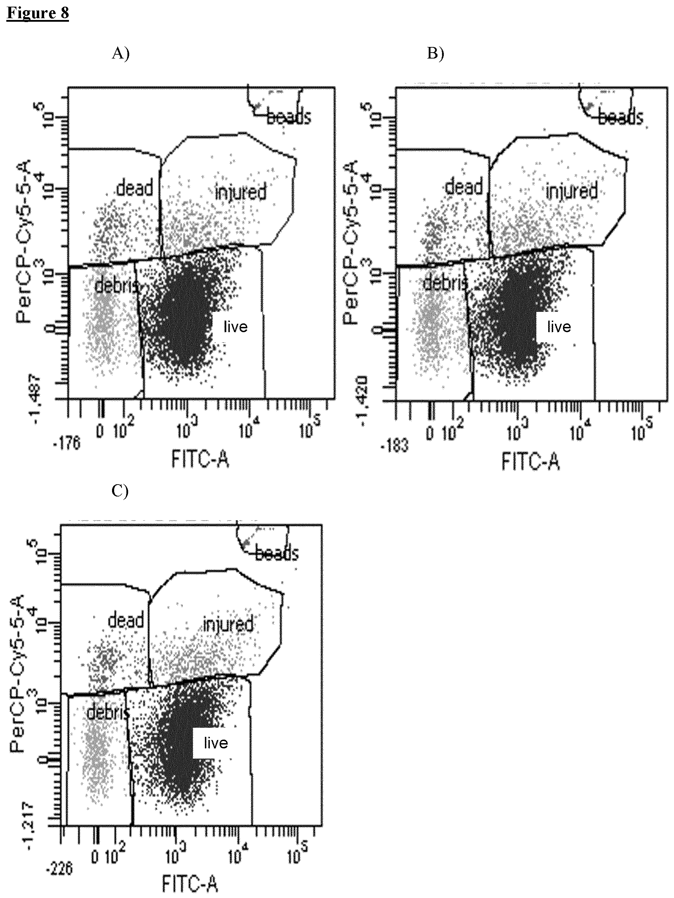

**Figure** 8: Flow Cytometry dotplots illustrating live cells present in encapsulated lactobacillus englobed material after exposure to beverage pre heat temperatures of 90°C (A). Live:dead cell proportions after exposure to full UHT heat process at 138°C for 6 seconds (B). Live: dead cell proportions after exposure to full UHT heat process at 143°C for 3 Seconds (C).

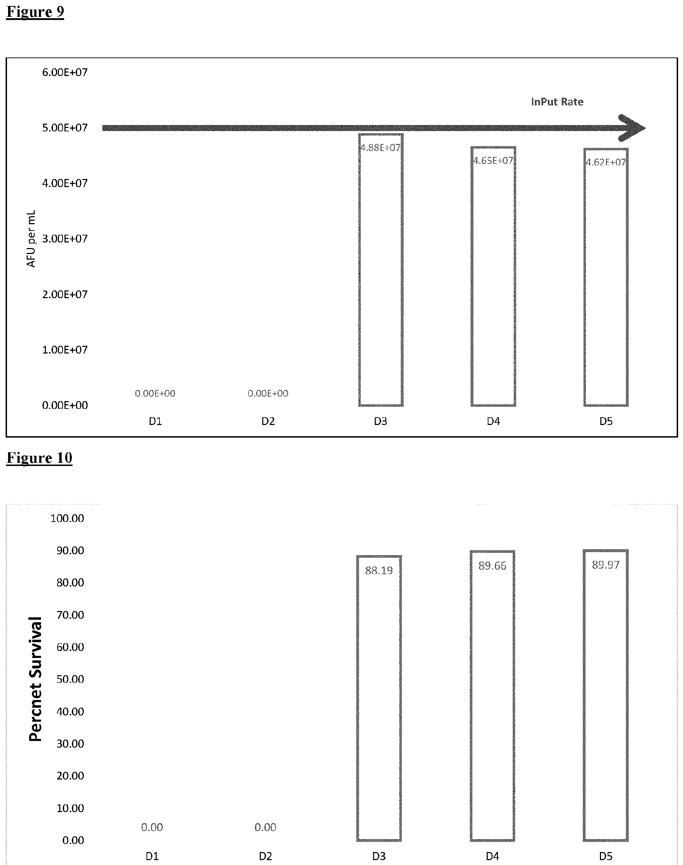

**Figure 9** is a block diagram illustrating probiotic survival in UHT treated protein beverage fortified with spray englobed microparticles with heat treatment of at 138 for 6 seconds. Data shows storage stability at 21 °C. Data illustrates cell survival per ml. Codes D1 represents a blank beverage control. D2 represents free cell control beverage (no survival in absence of encapsulation).
**Figure 10** is a block diagram illustrating probiotic survival in UHT treated protein beverage fortified with spray englobed microparticles with heat treatment of at 138 for 6 seconds. Data shows storage stability at 21 °C. Data illustrates % survival after UHT heat processing. Codes D1 and D2 represent free cell controls.

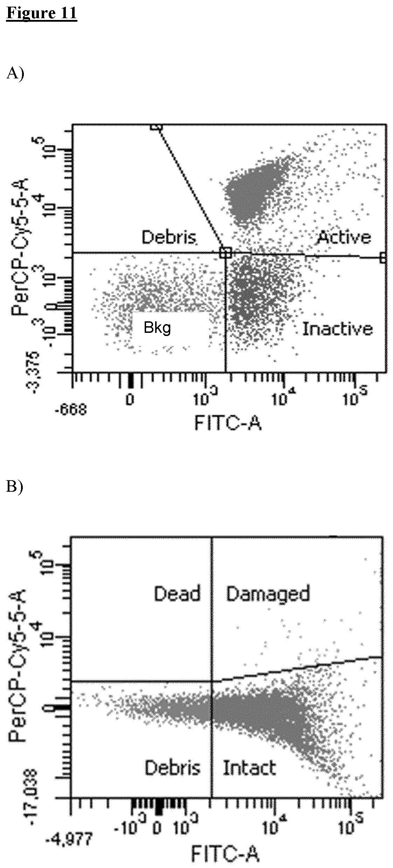

**Figure 11** is a block diagram illustrating probiotic cell functionality in UHT treated protein beverage fortified with spray englobed microparticles. Data shows probiotic storage stability at 21°C after extended storage (3 months). Data illustrates cFDA metabolic activity (A) and DioC2 membrane integrity (B) of Lactobacillus after UHT processing in dairy beverage with 3-month storage stability.

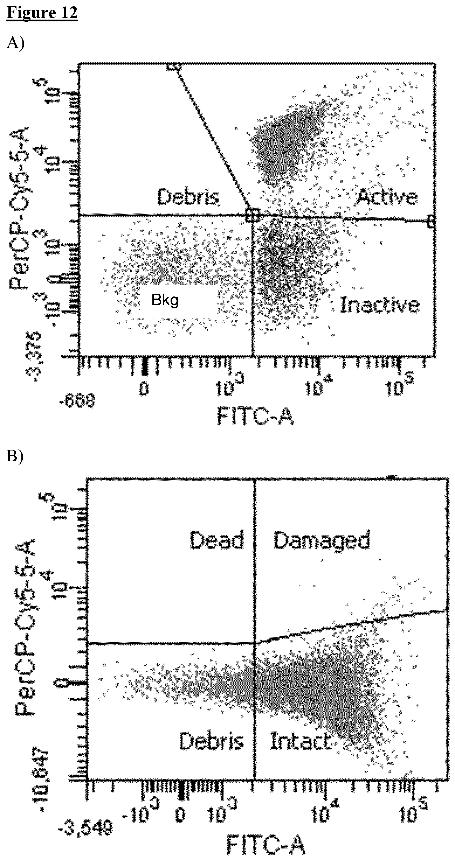

**Figure 12** is a block diagram illustrating probiotic cell metabolism in UHT treated protein beverage fortified with spray englobed microparticles. Data shows probiotic storage stability at 21°C after extended storage. Data illustrates cFDA metabolic activity (A) and DioC2 membrane integrity (B) of Bifidobacteria after UHT processing in dairy beverage with 3 month storage stability.

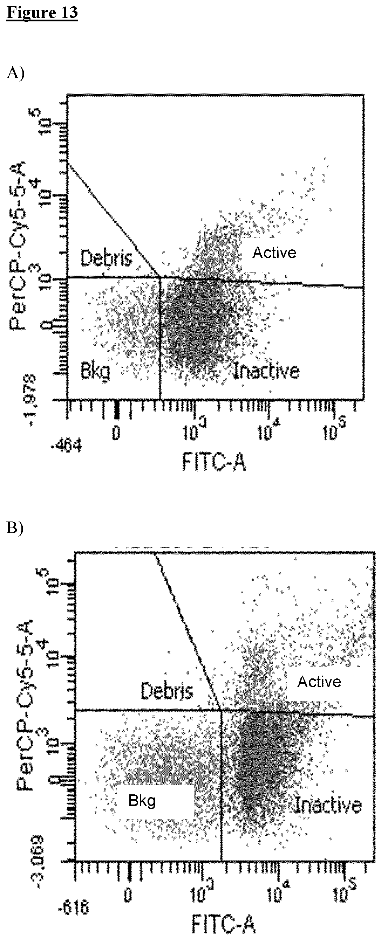

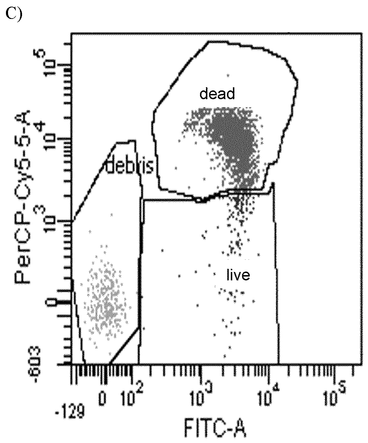

**Figure 13** is a block diagram illustrating probiotic cell metabolism (A) and membrane integrity (B) of free lactobacillus probiotics after pre heat (50°C) of a protein beverage. Data shows total probiotic death and injury in UHT beverage after UHT treatment (C).

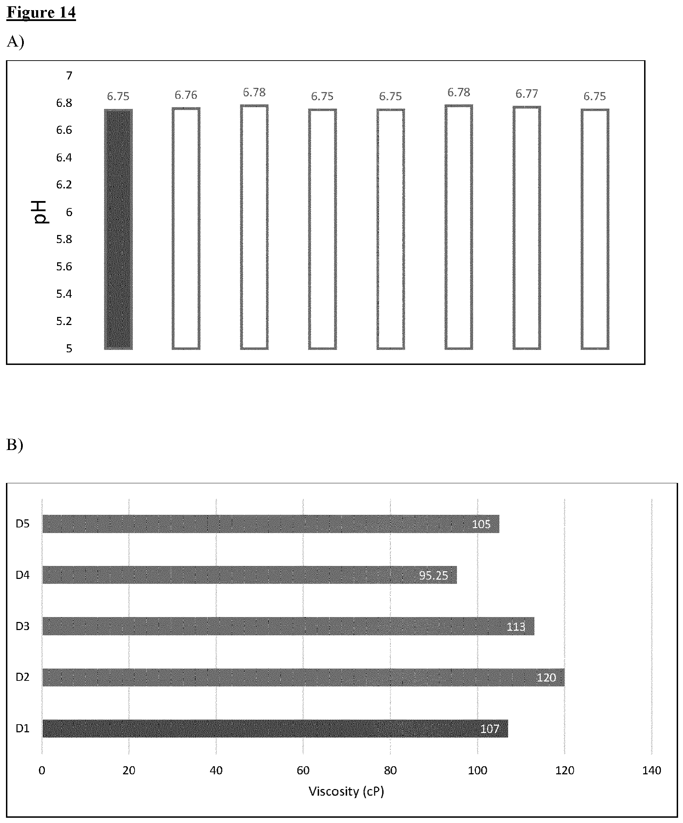

**Figure 14** is a block diagram illustrating UHT treated protein beverage physicochemical properties during storage stability after fortification with spray englobed microparticles. (A) shows the pH values of UHT Protein beverage after extended storage at 37°C fortified with spray englobed microparticles. Red column represents control UHT beverage with no probiotics. (B) the viscosity values of UHT Protein beverage after extended storage at 37°C fortified with spray englobed microparticles. Red column represents control UHT beverage with no probiotics.

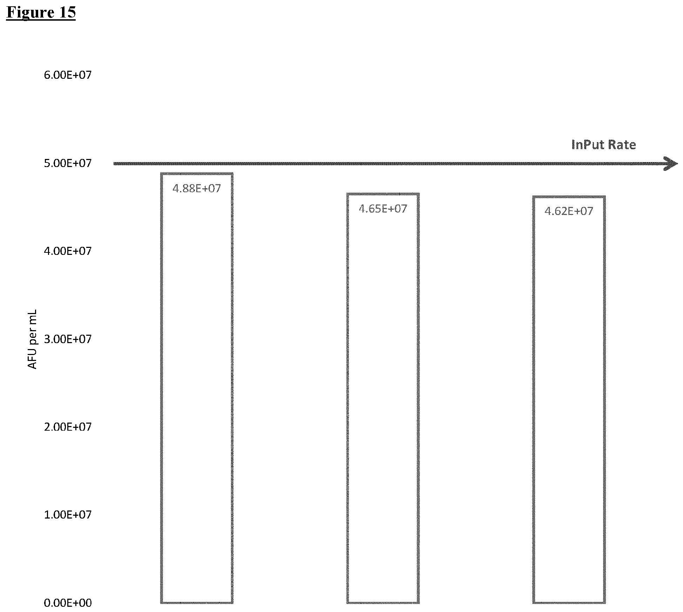

**Figure 15** is a diagram illustrating UHT treated protein beverage fortified with englobed Lactobacillus microparticles in 3 independent trials. Data illustrates the reproducibly high cell survival post UHT treatment at 143°C for 3 seconds.

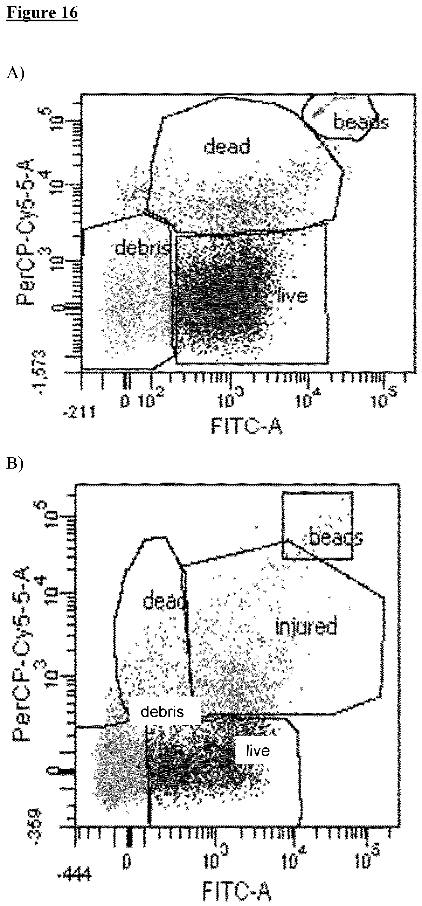

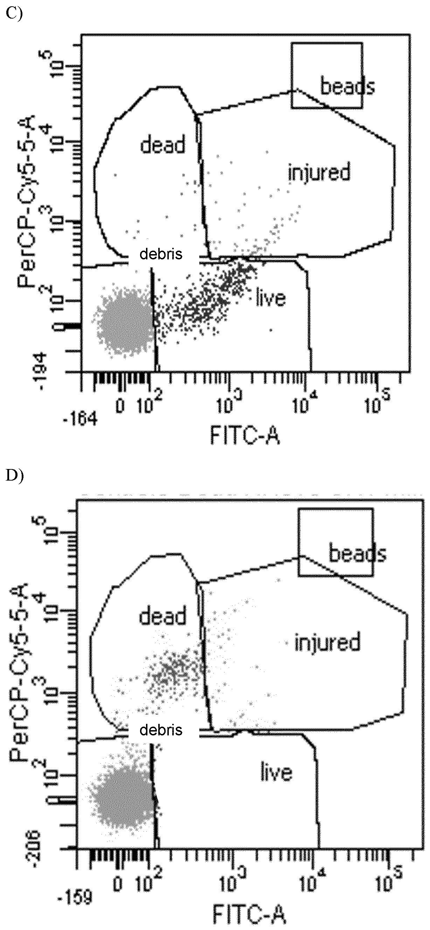

**Figure 16** is a diagram illustrating UHT treated protein beverage after extended month storage. Data illustrates verified total cell survival post UHT treatment and 3-month storage. Data shows UHT beverage with dose of probiotics at 10⁸ probiotics per ml (A); UHT beverage with dose of probiotics at 10⁷ probiotics per ml (B); UHT beverage with dose of probiotics at 10⁶ probiotics per ml (C). Control beverages prepared with free cells show fully dead population (D).

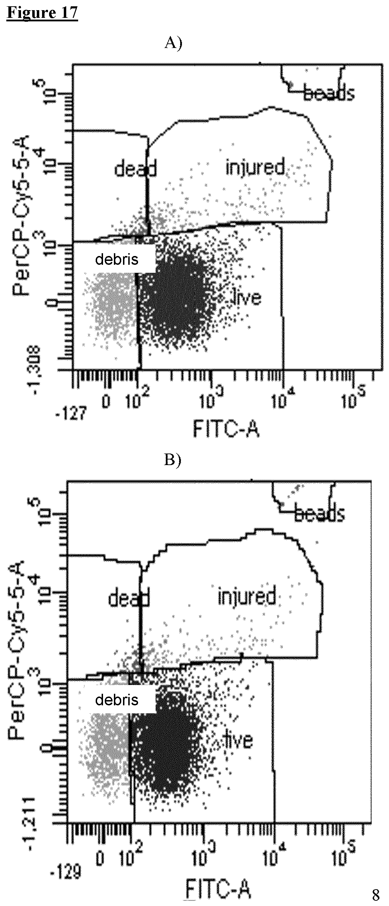

**Figure 17** is a diagram illustrating UHT treated protein beverage after extended month storage and subjected to full human in vitro digestion. Data illustrates the release of *Lactobacillus* (A) and *Bifidobacteria* (B) from respective UHT beverages after full human in vitro digestion.

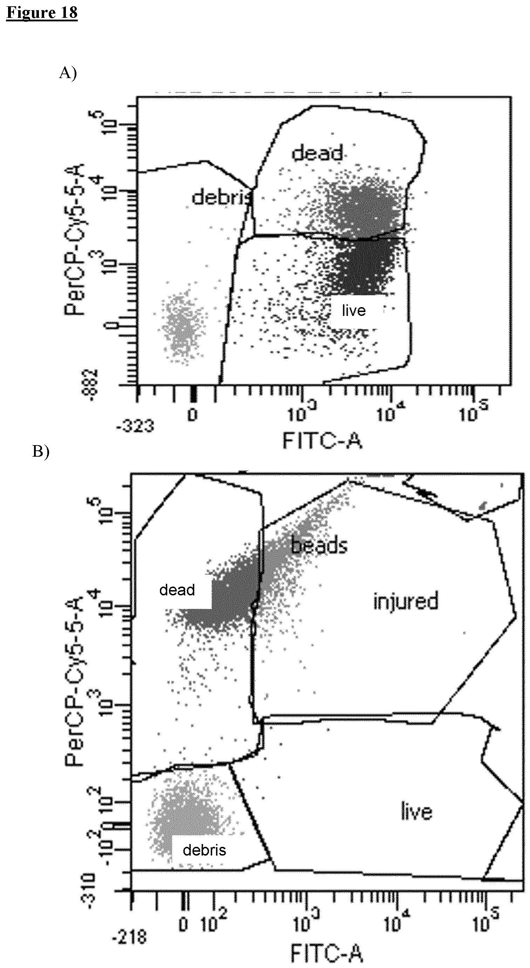

**Figure 18** is a diagram illustrating free probiotic cells exposed to full human in vitro digestion. Data illustrates the injury and death of lactobacillus after stomach incubation (A) and full cell injury and degradation after intestinal incubation (B).

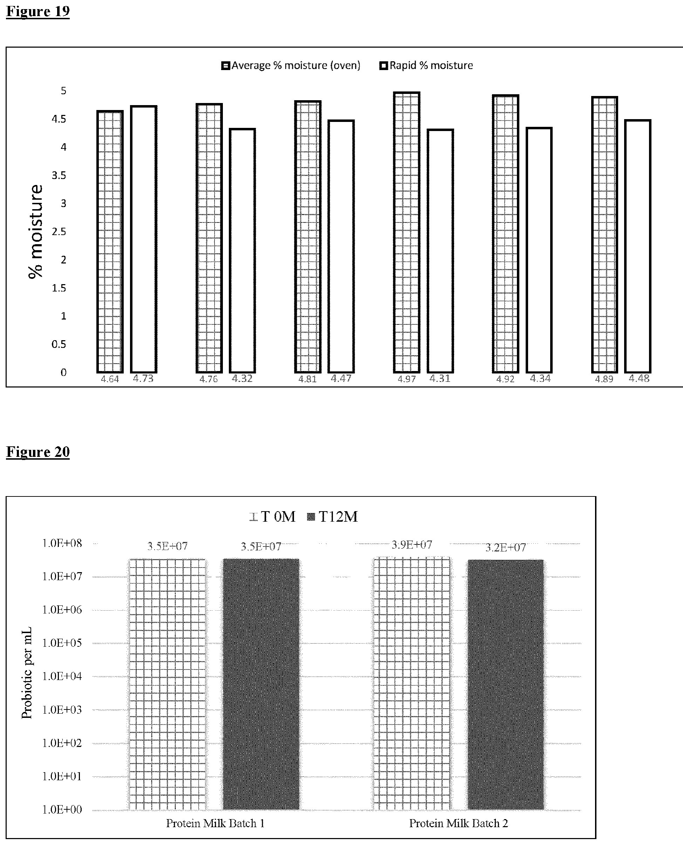

**Figure 19** illustration of moisture values of across multiples batches of spray englobed microparticle
**Figure 20****:** Probiotic stability data after storage for 12 months at 25°C from High Protein Milk UHT (140°C - 6 seconds), fortified with Encapsulated *Bifidobacterium Lactis* using denatured Mung Bean Protein. Data presented for Time 0 and Time 12 months stability, for duplicates batches 1 and 2.

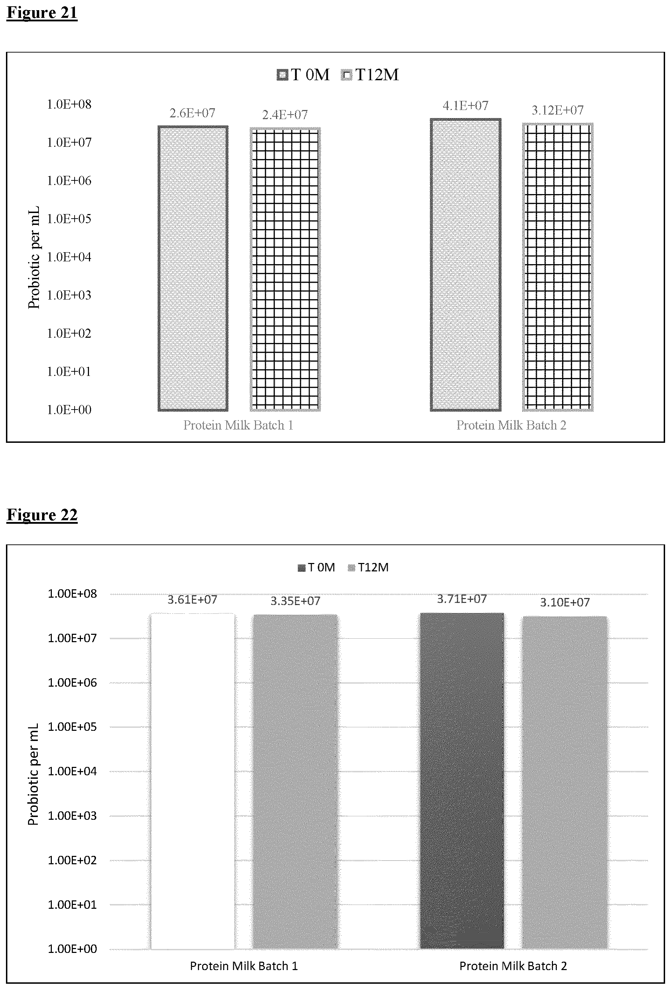

**Figure 21****:** Probiotic stability data after storage for 12 months at 25°C from High Protein Milk UHT (140°C - 6 seconds), fortified with Encapsulated *Bifidobacterium Lactis* using denatured Pea Protein. Data presented for Time 0 and Time 12 months stability, for duplicates batches 1 and 2.
**Figure 22****:** Probiotic stability data after storage for 12 months at 25°C from High Protein Milk UHT (140°C - 6 seconds), fortified with Encapsulated *Lb Rhamnosus* using denatured Pea Protein. Data presented for Time 0 and Time 12 months stability, for duplicates batches 1 and 2.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "heat treated" as applied to the liquid product should be understood to mean at least a pasteurisation treatment of the liquid product and, in some embodiments Ultra Pasteurisation (UP) or a sterilisation process such as Ultra Heat Treatment (UHT). Pasteurisation of liquid beverages is a well-known and understood process that involves heating a liquid product for a defined time and temperature to kill pathogens in the product. High temperature Short Time (HTST) pasteurisation involves heating the liquid product at 71.5°C to 74°C for 15 to 30 seconds, although other temperature-time combinations are possible depending on the product. Often the process involves rapid cooling to ambient or refrigerated temperatures. UHT involves processing a liquid product at high temperatures (e.g. about 135°C - 154°C) for short holding times (e.g. about 1-2 second), with a view to eliminating microbial spores from the product to give it a longer shelf-life. Another pasteurization treatment resulting in long-life products is HHST (higher heat/shorter term) which lies in between HTST and UHT in terms of time and temperature. UHT treated liquid products may be stored for extended periods at ambient temperature.

As used herein, the term "shelf-stable" as applied to a liquid product means a liquid product that has a shelf life of at least 6 months when stored at ambient temperature (21°C). In any embodiment, the liquid product has a shelf life of at least 7, 8, 9, 10, 11 or 12 months when stored at ambient temperature (21°C).

As used herein, the term "low acid beverage" refers to any beverage that has a pH of more than 4.6, for example water beverages, herbal beverages, dairy beverages, non-dairy beverages, non-fermented beverages.

As used herein, the term "high acid beverage" refers to a beverage that has a pH less than 4.6, for example, fermented beverages, fruit juices, vegetable juices, non-dairy-beverages.

As used herein, the term "spray englobed microparticles" refers to microparticles formed in a fluidised bed process and having a core containing an active agent such as a probiotic and a carrier material and having a denatured protein coat enrobing the core and protecting the active agent from heat treatment. The core generally comprises an agglomerate of sub-microparticles, optionally bound together with a binder. The core may include a glidant or a lubricant. The coating may contain a glidant. The agglomerate is generally formed in a fluidised bed, usually comprising a first stage in which active agent and carrier material are fluidised to produce sub-microparticles which then agglomerate, typically with a binder that is optionally applied by top spraying. In the second stage, the coating is applied subsequently in a bottom spraying (Wurster) fluidised bed and optionally a polymerisation salt is bottom sprayed on to the coated microparticles, generally before the coating has dried.

Some examples of the process of the invention are provided below:

### Probiotic Spray Englobing using denatured protein dPPI

- Prepare dry ingredients and load into chamber.
- Prepare denatured protein suspension.
- Begin fluidisation process and activate heating (35 - 45 °C).
- Fluidise with airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture.
- Re-commence spray englobing process using maltodextrin (15% w/v solution).
- Apply interim drying step to reduce moisture by 25%.
- Re-commence spray englobing process using denatured protein and conduct 2 - 3 cycles of spray englobing.
- Implement a final post dry step to generate final moisture content below 5%.

### Probiotic Spray Englobing using denatured pea protein dPPI and calcium cation cross-linker.

- Prepare dry ingredients (protein, probiotic and simple sugar) and load into chamber.
- Prepare denatured protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the calcium cross-linker.
- Implement a final post dry step to generate final moisture content below 5%.

### Probiotic Spray Englobing using denatured mung protein and calcium cation cross-linker

- Prepare dry ingredients and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture.
- Re-commence spray englobing process using Calcium chloride (0.2- 0.4 M)
- Apply interim drying step to reduce moisture by 25%.
- Re-commence spray englobing process using denatured protein and conduct 2 - 3 cycles of spray englobing using denatured protein and the calcium or magnesium cation cross-linker.
- Implement a final post dry step to generate final moisture content below 5%.

### Probiotic Spray Englobing using denatured mung protein, high oleic coconut oil and calcium cation cross-linker.

- Prepare dry ingredients and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using oil, maltodextrin and / or denatured protein.
- Nozzle Pressure should be maintained between 2 - 4.5 Bar nozzle pressure air flow adjusted accordingly.
- Apply interim drying step to reduce moisture.
- Re-commence spray englobing process using high oleic coconut oil.
- Apply interim drying step to reduce moisture.
- Re-commence spray englobing process.
- Apply interim drying step to reduce moisture.
- Re-commence spray englobing process using Calcium chloride (0.2 - 0.4 M)
- Apply interim drying step to reduce moisture.
- Re-commence spray englobing process using denatured protein and conduct 2 - 3 cycles of spray englobing using denatured protein and the calcium cation cross-linker.
- Implement a final post dry step to generate final moisture content below 5%.

As used herein, the term "bottom spraying" refers to the method of coating the agglomerates with coating material (e.g. denatured protein) that employs a bottom spraying nozzle to fluidise the agglomerates, and spray the coating material on to the agglomerates, from below. This process generally employs a fluidised bed incorporating a Wurster insert which allows a mixture of air and coating material to be sprayed onto the agglomerates from below. Wurster fluidised beds and inserts are described in Kolaf et al. (Powder Technology, Vol 386,m July 2021) and Mohylyuk et al. (AAPS PharmSciTech. 2020 Jan; 21(1): 3).

As used herein, the term "sub-microparticles" refers to particles that are smaller than the spray englobed microparticle and which comprise active agent (e.g., live probiotic) and carrier material, and optionally a binder such as lipid or denatured protein. The sub-microparticles generally have an average dimension of less than 30%, 20% or 10% of the average dimension of the spray englobed microparticles.

As used herein, the term "carrier material" refers to a food grade carrier material for the active agent, for example a native protein, a carbohydrate such as maltodextrin or starch, a prebiotic fibre powder or any colloid, or any combination thereof. In one embodiment, the carrier comprises carbohydrate and native food grade protein. The native food grade protein is generally a plant protein, for example pea or mung bean protein. Other plant proteins suitable for use as the carrier are described herein.

As used herein, the term "binding agent" refers to a food grade material that may be added to core of the spray englobed microparticle to help glue the active agent and carrier material together and produce the agglomerate of sub-microparticles. Examples include denatured protein and lipid. The lipid may be solid or liquid (e.g. an edible oil). When the binder is denatured protein, a polymerising salt may be added to polymerise the protein.

As used herein, the term "dry" as applied to the spray englobed microparticles means a water activity (AW) of less than 0.4. Typically, the dry spray englobed microparticles have a water activity (AW) of less than 0.3 or 0.2.

As used herein, the term "plant protein" refers to a protein preparation obtained from a plant source. Examples include plant protein powders, plant protein concentrates and plant protein isolates. Plant protein preparations can be obtained from pea, zein, barley, spelt, soya, seaweed, hemp, seiten (wheat gluten), chickpea, tofu, lentil, mung bean, quinoa and other plant sources. Plant proteins having an isoelectric point above pH 5, 5.5 or 6 are preferred. The plant protein isolates may be denatured pea protein (dPPI) or ultra-denatured pea protein isolate (udPPI), manufactured according to Examples 1 and 2 of WO 2023/144354

As used herein, the term "sugar", "carbohydrate" or "simple carbohydrate" includes monosaccharides such as glucose, fructose, galactose and disaccharides such as sucrose, lactose and maltose, and maltodextrin, and mixtures thereof. In one embodiment, the simple carbohydrate comprises maltodextrin / dextrose . Maltodextrin may be provided in the form of corn syrup solids, modified corn starch, modified rice starch, modified tapioca starch, modified wheat starch, modified potato starch.

As used herein, the term "polymerisation agent" refers to an agent that polymerises denatured food grade protein. Examples include divalent metal salts selected from calcium, iron and zinc salts, and weak acids such as citric acid. Preferably the polymerisation agent is calcium chloride.

The microparticles of the invention may comprise a prebiotic, typically in the core of the microparticle. "Prebiotic" means food substances that promote the growth of probiotics in the intestines They are not broken down in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412. The prebiotics that may be used in accordance with the present invention are not particularly limited and include all food substances that promote the growth of probiotics in the intestines. Preferably, they may be selected from the group consisting of oligosaccharides, optionally containing fructose, galactose, mannose; dietary fibres, in particular soluble fibres, soy fibres; inulin; or mixtures thereof. Preferred prebiotics are fructo-oligosaccharides (FOS), galacto-oligosaccharides (IOS), isomalto-oligosaccharides, xylo-oligosaccharides, oligosaccharides of soy, glycosylsucrose (GS), lactosucrose (LS), lactulose (LA), palatinose-oligosaccharides (PAO), malto-oligosaccharides (MOS), gums and/or hydrolysates thereof, pectins and/or hydrolysates thereof. Typical examples of prebiotics are oligofructose and inulin. The quantity of prebiotics in a beverage depends on their capacity to promote the development of lactic acid bacteria upon reaching the intestine. Addition of prebiotic substances can be added to agglomeration / top spray stage. In the products of the invention the advantages of probiotics (provided in the form of spores) can be further broadened by the addition of prebiotic substances. Prebiotics substances are non-digestible food ingredients that beneficially affect the host by selectively stimulating the growth and/or activity of one or a limited number of bacteria in the intestines (notably the colon), and thus improve host health. The best-known prebiotic substances are non-digestible oligosaccharides. Prebiotics are fructo and/or galacto-oligosaccharides, with short or long chains, inulin, fucose-containing oligosaccharides, beta glycans, carob flour, gums, pectins, sialyloligosaccharides, sialyllactose, galactans with short or long chains, and nucleotides. Regular amounts will be 0.1-10 grams, preferably 1-5 grams, or 2-3 grams a day dependent on the prebiotic substances employed.

As used herein, the term "probiotic" or "live probiotic" refers to live beneficial bacteria and/or yeasts that naturally live in your body. Probiotics help keep your body healthy and working well, including fighting off bad bacteria when you have too much of it, helping you feel better. The probiotics employed in the invention may comprise a combination of different probiotics. Though there are many types of bacteria that can be considered probiotics, there are two specific types of bacteria that are common probiotics found in stores. These include *Lactobacillus* and *Bifidobacterium.* In any embodiment, the probiotic is a non-spore forming bacterium. Examples of commercially available probiotic bacteria include *Lactobacillus rhamnosus* GG, *Bifidobacterium lactis, Bifidobacterium infantis 35624, Lactobacillus* GG, *Lactobacillus rhamnosus* GR-1, *Lactobacillus reuteri* RC-14 and *Lactobacillus acidophilus* LA-5.The probiotic is generally a vegetative (non-spore-forming) bacterium although the invention may also be applied to spore forming bacteria such as Bacillus.

As used herein, the term "% probiotic survival" as applied to the compositions made according to the method of the invention means the % of live cells that survive the encapsulation process (including the drying process where the process includes a drying step). % probiotic survival can be measured by determining the live cell input into the process and comparing with the number of cells in the output product.

As used herein, the term "active agent" refers to any component suitable for delivery to the mammalian small intestine or ileum, but typically means a component that is sensitive to an external condition for example heat, pH, moisture, pressure, chemical stress or enzymes. Thus, the active component may be sensitive to one or more of pH, enzymes (i.e. protease enzymes), high pressure, moisture, high shear, and temperature abuse during storage. In one particularly preferred embodiment of the invention, the active agent is heat-sensitive, such as a bacterium, some micronutrients, caffeine, fat soluble vitamins (A, D, E, K), water soluble vitamins (B and C), hyaluronic acid, melatonin, and marine bioactives such as fucoidan and laminarin, green, brown and red seaweed extract.

"Gastric-resistant": means that the microparticles can survive intact for at least 60 minutes in the simulated stomach digestion model described in Minekus et al., 1999 and 2014 (A computer-controlled system to simulate conditions of the large intestine with peristaltic mixing, water absorption and absorption of fermentation product, Minekus, M., Smeets-Peeters M, Bernalier A, Marol-Bonnin S, Havenaar R, Marteau P, Alric M, Fonty G, Huis in't Veld JH, Applied Microbiology Biotechnology. 1999 Dec;53 (1):108-14) and (Minekus et al., 2014, A standardised static in vitro digestion method suitable for food - an international consensus, Minekus, A. et al., Food Function, 2014, 5, 1113).

"Ileal-sensitive": means that the microparticles are capable of releasing their contents in vivo in the ileum (proximal or distal) of a mammal.

"Viable" as applied to the probiotic bacteria in the microparticles of the invention after storage means that the cells are physiologically active and capable of conferring a benefit to the consumer. The benefit may be one of the health benefits mentioned above, or it may mean that the cells are capable of colonising the gut of the consumer and acting as a competitive deterrent to colonisation of the gut by pathogenic bacteria. Viability of probiotic bacteria in the compositions and beverages of the invention are determined quantitatively by a combination of flow cytometry and FACS using the ISO 19344 FACS protocols ( ISOO 19344 | IDF 232: 2015) that generates information about probiotic functional status and cellular activities during shelf-life and storage of UHT beverages fortified with probiotic bacteria. Flow cytometry combines the flexibility and sensitivity of fluorescence technology with high speed and data integration capabilities to support detailed quantification of probiotic cells in UHT beverages. The ISO 19344 protocol can be used as an analytic tool to assess encapsulated probiotic function and viability in various beverage applications including UTH ambient beverages.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1

*Probiotic Spray Englobing using denatured plant protein dPPI* / *udPPI*
- Prepare dry ingredients and load into chamber (protein, probiotic and simple sugar).
- Prepare denatured protein suspension.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using maltodextrin (15% w/v solution).
- Apply interim drying step to reduce moisture by 25%.
- Re-commence spray englobing process using denatured protein and conduct 2 - 3 cycles of spray englobing using denatured protein and the simple carbohydrate solution (maltodextrin).
- Implement a final post dry step to generate final moisture content below 5%.

### EXAMPLE 2

*Probiotic Spray Englobing using denatured pea protein dPPI*/ *udPPI and calcium cation cross-linker*
- Prepare dry ingredients (protein, probiotic and simple sugar) and load into the chamber.
- Prepare denatured protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly
- Apply interim drying step to reduce moisture by 50%.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the calcium cross-linker.
- Implement a final post dry step to generate final moisture content below 5%.

### EXAMPLE 3

*Probiotic Spray Englobing using denatured pea protein dPPI*/ *udPPI , high oleic coconut oil and calcium cation cross-linker.*
- Prepare dry ingredients (protein, probiotic and simple sugar) and load into chamber.
- Prepare denatured pea protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using high oleic coconut oil.
- Apply interim drying step to reduce moisture by 25%.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the calcium or magnesium cation cross-linker.
- Implement a final post dry step to generate final moisture content below 5%.

### EXAMPLE 4

*Probiotic Spray Englobing using denatured mung protein and calcium cation cross-linker.*
- Prepare dry ingredients (protein, probiotic and simple sugar) and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using Calcium chloride (0.2- 0.4 M).
- Apply interim drying step to reduce moisture by 25%.
- Re-commence spray englobing process using denatured protein and conduct 2 - 3 cycles of spray englobing using denatured protein and the calcium or magnesium cation cross-linker.
- Implement a final post dry step to generate final moisture content below 5%.

### EXAMPLE 5

*Probiotic Spray Englobing using denatured mung protein, high oleic sunflower oil and calcium cation cross-linker.*
- Prepare dry ingredients (protein, probiotic and simple sugar) and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured plant protein.
- Nozzle Pressure should be maintained between 2 - 4.5 Bar nozzle pressure air flow adjusted accordingly.
- Apply interim drying step to reduce moisture by 50%.
- Re-commence spray englobing process using high oleic sunflower oil.
- Apply interim drying step to reduce moisture by 25%.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the calcium or magnesium cation cross-linker.
- Implement a final post dry step to generate final moisture content below 5%.

### EXAMPLE 6

*Probiotic Spray Englobing using denatured mung protein, high oleic coconut oil and calcium cation cross-linker.*
- Prepare dry ingredients (protein, probiotic and simple sugar) and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using high oleic coconut oil.
- Apply interim drying step to reduce moisture by 25%.
- Re-commence spray englobing process using high oleic sunflower oil.
- Apply interim drying step to reduce moisture by 25%.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the calcium cation cross-linker.
- Implement a final post dry step to generate final moisture content below 5%.

### EXAMPLE 7

*Probiotic Spray Englobing using denatured pea protein dPPI*/ *udPPI , high oleic coconut oil.*
- Prepare dry ingredients (protein, probiotic) and load into chamber.
- Prepare denatured mung protein suspension and calcium cation cross-linker.
- Begin fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using high oleic coconut oil.
- Apply interim drying step to reduce moisture by 25%.
- Re-commence spray englobing process using simple carbohydrate (maltodextrin).
- Apply interim drying step to reduce moisture by 25%.
- Re-commence spray englobing process using denatured protein and conduct 2 - 3 cycles of spray englobing using denatured protein and the simple carbohydrate solution.
- Implement a final post dry step to generate final moisture content below 5%.

### EXAMPLE 8

*Probiotic Spray Englobing using denatured mung protein and calcium cation cross-linker with optimised coating layer.*
- Prepare dry ingredients (protein, probiotic) and load into chamber.
- Prepare denatured mung protein suspension and calcium cation cross-linker.
- Begin top -spray fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using Calcium chloride (0.2 - 0.4M)
- Apply interim drying step to reduce moisture by 25%.
- Re-commence spray englobing process using denatured protein and conduct 2 - 3 cycles of spray englobing using denatured protein and the magnesium cation cross-linker.
- Implement a final post dry step to generate final moisture content below 5%.
- Initiate bottom spray englobing using denatured mung bean protein.
- Utilised nozzle pressure in excess of 2 Bar.
- Maintain accelerator air pressure above 1.5 Bar during spray englobing.
- Maintain airflow at equilibration (approx. 90 - 170 m³/hr).
- Maintain product temperature between 37 - 45 °C.
- Apply intermittent post drying at 45 - 55 °C.
- Implement at least 2 cycles of calcium cross-linking spray englobing steps with subsequent intermittent drying to achieve 25% reduction on moisture content.
- Apply final post dry step to achieve final moisture content below 5%.

### EXAMPLE 9

*Probiotic Spray Englobing using denatured pea protein, high oleic coconut oil and calcium cation cross-linker with optimised coating layer.*
- Prepare dry ingredients (protein, probiotic) and load into chamber.
- Prepare denatured mung protein suspension and calcium cation cross-linker.
- Begin top-spray fluidisation process and activate heating (35 - 45 °C).
- Fluidise with an airflow of 110 - 300 m³/hr with set air inlet temperatures.
- Once product temperature is equilibrated, commence spray englobing process using denatured protein.
- Nozzle Pressure should be maintained between 2 - 4.5 Bar nozzle pressure air flow adjusted accordingly.
- Apply interim drying step to reduce moisture by 50%.
- Re-commence spray englobing process using high oleic coconut oil.
- Apply interim drying step to reduce moisture by 25%.
- Re-commence spray englobing process using Calcium chloride (0.2 - 0.4 M)
- Apply interim drying step to reduce moisture by 25%.
- Re-commence spray englobing process using denatured protein and conduct 2 - 3 cycles of spray englobing using denatured protein and the magnesium cation cross-linker.
- Implement a final post dry step to generate final moisture content below 5%.
- Initiate bottom spray englobing using denatured protein.
- Utilised a nozzle pressure in excess of 2 Bar.
- Maintain accelerator air pressure above 1.5 Bar during spray englobing.
- Maintain airflow at equilibration (approx 90 - 170 m³/hr)
- Maintain product temperature between 37 - 45 °C.
- Apply intermittent post drying at 45 - 55 °C.
- Apply spray englobing process using high oleic coconut oil.
- Apply interim drying step to reduce moisture by 25%.
- Implement at least 2 cycles of calcium cross-linking spray englobing steps with subsequent intermittent drying to achieve 25% reduction on moisture content.
- Apply final post dry step to achieve final moisture content below 5%.

### EXAMPLE 10

*Spray englobing of probiotics, bioactives in denatured dPPI or udPPI*
- Using dPPI prepared as per Example 1, prepare a suspension of dPPI /udPPI and agitate at 250 RPM at RT.
- Add 2% glucidex / glucose / simple carbohydrate to the protein solution prior to encapsulation (this adds charge to the denatured protein solution and creates a compatible matrix for probiotics).
- Hydrate at room temperature for 2 hours using mild agitation to avoid creation of air pockets.
- Load fluidised bed chamber with dry pea protein powder (native powder; approx. 2% moisture).
- Fluidise at 37 °C at high airflow.
- Added probiotic culture to the powder at 5:1 or 10:1 ratio.
- Add 15 Calcium Citrate or Calcium Chloride to the chamber and fluidise.
- Spray 10% glucidex solution onto the material at high flow rate (20-30 RPM) high airflow(>150 RPM) and low nozzle pressure (<1.5 bar) promotes agglomeration.
- Spray coconut oil / sunflower oil / high oleic oil onto the powder at low flow rate (18-22 RPM), high airflow(>150 RPM) and high nozzle pressure (> 2.5 bar).
- Spray dPPI - glucidex solution into chamber at high flow rate (25-30 RPM), high airflow(>150 RPM) and high nozzle pressure (> 2.5 bar).
- Post dry the material to achieve Aw < 0.15.

### EXAMPLE 11: Pasteurisation of a beverage

- *Hydrate beverage ingredients using sequential formulation principles.*
- *Apply high shear to fully hydrate the fibre and plant protein materials.*
- *If milk protein is present, temperature of hold vessel should not* excess *70°C.*
- *Equilibrate the suspension for 30 min.*
- *Add flavours, stabilisers and micronutrients using in-line blending apparatus.*
- *Add spray-englobed probiotic microparticles (min 1x10⁶ CFU per ml) using low shear and equilibrate the suspension for min 15 min.*
- *Transfer the suspension to a hold tank with controlled temperature and continued agitation.*
- *Initiate pasteurisation heat process at min 75 °C with min* 1 sec *hold time.*
- *Cool beverage suspension to 22- 35 °C and aseptically fill into containers.*

### EXAMPLE 12: Single Homogenisation + Pasteurisation + Single Homogenisation

- *Hydrate beverage ingredients using sequential formulation principles.*
- *Apply high shear to fully hydrate the fibre and plant protein materials.*
- *If milk protein is present, temperature of hold vessel should not* excess *70°C.*
- *Equilibrate the suspension for min 30 min.*
- *Add flavours, stabilisers and micronutrients using in-line blending apparatus.*
- *Transfer the suspension to a hold tank with controlled temperature and continued agitation.*
- *Homogenise the suspension using single extrusion homogenisation.*
- *Hold the suspension at 30°C until viscosity and pH are equilibrated.*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Initiate Pasteurisation at min 75°C with min* 1 sec *hold time*
- *Homogenise the suspension (2^{nd} stage homogenisation) using single extrusion.*
- *Hold the suspension at 25°C and aseptically fill into containers.*

### EXAMPLE 13: Double Homogenisation + Pasteurisation + UHT treatment of a Fermented beverage

- *Hydrate beverage ingredients using sequential formulation principles.*
- *Apply high shear to fully hydrate the fibre and plant protein materials.*
- *Equilibrate the suspension for min 30 min.*
- *Add flavours, stabilisers and micronutrients using in-line blending apparatus*
- *Add starter culture suspension to initiate fermentation i.e. kombucha, yoghurt, kefir beverage suspensions would involve the use* of some *of the following strains;* Lactobacillus acidophilus, Lactobacillus delbrueckii ssp.bulgaricus, Bifidobacterium bifidum / Streptococcus thermophilus / L. kefiranofacien / Symbiotic culture of bacteria and yeast (SCOBY)
- *Transfer the suspension to a hold tank with controlled temperature and continued agitation.*
- *Once fermentation is complete (based on time or acid generation* / *pH measurement), homogenise the suspension using double extrusion homogenisation.*
   *Hold the suspension at 30 °C* Core of active agent (e.g. probiotic) and carrier material
   Inner coating of polymerised denatured plant protein
   Outer coating of polymerised denatured plant protein
   First intermediate coating of oil.
   Second intermediate coating of non-polymerised dried denatured plant protein
- *egC until viscosity and pH is equilibrated*
- *Pasteurise the suspension at min 75 °C with min 3 sec hold time*
- *Cool beverage suspension to 30 °C.*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Initiate UHT heat process at min 121°C with min 1 sec hold time.*
- *Cool beverage suspension to 22- 35°C and aseptically fill into containers.*

### EXAMPLE 14 Pasteurisation + Single Homogenisation + UHT treatment of Beverage + Single Homogenisation

- *Hydrate beverage ingredients using sequential formulation principles.*
- *Apply high shear to fully hydrate the fibre and plant protein materials.*
- *If milk proteins are present, temperature of hold vessel should not* excess *70°C.*
- *Equilibrate the suspension for min 30 min.*
- *Add flavours, stabilisers and micronutrients using in-line blending apparatus.*
- *Transfer the suspension to a hold tank with controlled temperature and continued agitation.*
- *Pasteurise the suspension at min 75°C with min* 3 sec *hold time.*
- *Cool beverage suspension to 30°C.*
- *Homogenise the suspension using single extrusion homogenisation.*
- *Hold the suspension at 30°C until viscosity and pH are equilibrated.*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Initiate UHT heat process at min 138°C with min 3 sec hold time.*
- *Homogenise the suspension (2^{nd} stage homogenisation) using single extrusion.*
- *Hold the suspension at 25°C and aseptically fill into containers.*

### EXAMPLE 15: Single Homogenisation + UHT treatment of Beverage + Single Homogenisation

- *Hydrate beverage ingredients using sequential formulation principles.*
- *Apply high shear to fully hydrate the fibre and plant protein materials.*
- *If milk proteins are present, temperature of hold vessel should not* excess *70°C.*
- *Equilibrate the suspension for min 30 min.*
- *Add flavours, stabilisers and micronutrients using in-line blending apparatus.*
- *Transfer the suspension to a hold tank with controlled temperature and continued agitation.*
- *Homogenise the suspension using single extrusion homogenisation.*
- *Hold the suspension at 30°C until viscosity and pH is equilibrated*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Initiate UHT heat process at min 138°C with min 3 sec hold time.*
- *Homogenise the suspension (2^{nd} stage homogenisation) using single extrusion.*
- *Hold the suspension at 25°C and aseptically fill into containers.*

### EXAMPLE 16: Single Homogenisation + Pasteurisation + UHT treatment of Beverage + Single Homogenisation

- *Hydrate beverage ingredients using sequential formulation principles.*
- *Apply high shear to fully hydrate the fibre and plant protein materials.*
- *If milk proteins are present, temperature of hold vessel should not* excess *70°C.*
- *Equilibrate the suspension for min 30 min.*
- *Add flavours, stabilisers and micronutrients using in-line blending apparatus.*
- *Transfer the suspension to a hold tank with controlled temperature and continued agitation.*
- *Homogenise the suspension using single extrusion homogenisation.*
- *Pasteurize the suspension at min 75°C for min 1 sec hold time.*
- *Cool the suspension to 25- 30°C.*
- *Transfer the suspension to a hold tank with controlled temperature and continued agitation.*
- *Add spray-englobed probiotic microparticles (min 1x10⁶ CFU per ml) using low shear and equilibrate the suspension for min 15 min until viscosity is steady (+*/ *50cPa).*
- *Initiate UHT heat process at min 138°C with min* 3 sec *hold time.*
- *Homogenise the suspension (2^{nd} stage homogenisation) using single extrusion.*
- *Hold the suspension at 25°C and aseptically fill into containers.*

### EXAMPLE 17: Double Homogenisation + Aseptic Filling + UHT treatment of Beverage

- *Hydrate beverage ingredients using sequential formulation principles.*
- *Apply high shear to fully hydrate the fibre and plant protein materials.*
- *If milk proteins are present, temperature of hold vessel should not* excess *70°C.*
- *Equilibrate the suspension for min 30 min.*
- *Add flavours, stabilisers and micronutrients using in-line blending apparatus.*
- *Transfer the suspension to a hold tank with controlled temperature and continued agitation.*
- *Homogenise the suspension using double extrusion homogenisation.*
- *Hold the suspension at 30°C until viscosity and pH are equilibrated.*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Aseptically fill into containers with agitation and controlled temperature*
- *Initiate UHT heat process at min 138°C with min 3 sec hold time.*
- *Cool to* 22 - *30°C.*

### EXAMPLE 18: Pasteurisation + double Homogenisation + packing + UHT treatment of a Fermented beverage

- *Hydrate beverage ingredients using sequential formulation principles.*
- *Apply high shear to fully hydrate the fibre and plant protein materials.*
- *Equilibrate the suspension for min 30 min.*
- *Add flavours, stabilisers and micronutrients using in-line blending apparatus.*
- *Add starter culture suspension to initiate fermentation i.e. kombucha, yoghurt, kefir beverage suspensions would involve the use* of some *of the following strains;* Lactobacillus acidophilus, *Lactobacillus delbrueckii ssp.bulgaricus, Bifidobacterium bifidum* / *Streptococcus thermophilus* / *L. kefiranofacien* / *Symbiotic* culture *of bacteria and yeast* (SCOBY)
- *Transfer the suspension to a hold tank with controlled temperature and continued agitation.*
- *Once fermentation is complete (based on time or acid generation* / *pH measurement), Pasteurise the suspension at min 75°C with min* 3 *sec hold time.*
- *Cool beverage suspension to 30°C.*
- *Homogenise the suspension using double extrusion homogenisation.*
- *Hold the suspension at 30°C until viscosity and pH are equilibrated.*
- *Add spray-englobed probiotic microparticles (min 1x10⁶ CFU per ml) using low shear and equilibrate the suspension for min 15 min until viscosity is steady (+*/ *50cPa).*
- *Transfer the suspension to a hold tank with controlled temperature and continued agitation.*
- *Aseptically fill containers using agitation and temperature control*
- *Initiate UHT heat process at min 138°C with min 3 sec hold time.*
- *Cool to 22- 30°C.*

**Table 1**

| *Physical properties across 12 month for Room Temperature stability and 6 months for accelerated stability at 37°C.* | | | | | | | | | | | | | | | | |
|---|---|---|---|---|---|---|---|---|---|---|---|---|---|---|---|---|
| | | | **Spoilage** | | | **Colour** | **Creaming** | | **Fat creep** | | **Sediment** | | **Serum** | | **Gelation** | |
| | **Code** | **Age (M)** | **Bloating** | **Curdling** | **Other** | | **Separation** | **post-dispersion** | **Fat creep** | **post-dispersion** | **Quiescent** | **post-dispersion** | **Separation** | **post-dispersion** | **Gelation** | **post-dispersion** |
| *Standard Product* | Room Temperature | 1 | 0 | 0 | - | light beige / cream colour | 0 | 0 | 0 | 0 | 0 | 0 | 0 | 0 | 0 | 0 |
| | Accelerrated (37DegC) | 1 | 0 | 0 | - | light beige / cream colour | 0 | 0 | 0 | 0 | 0 | 0 | 0 | 0 | 0 | 0 |
| *Probiotic UHT* | Room Temperature | 1 | 0 | 0 | - | light beige / sand colour | 0 | 0 | 0 | 0 | 0 | 0 | 0 | 0 | 0 | 0 |
| *Protein Milk* | Accelerrated (37DegC) | 1 | 0 | 0 | - | light beige / cream colour | 0 | 0 | 0 | 0 | 0 | 0 | 0 | 0 | 0 | 0 |
| | | | | | | | | | | | | | | | | |

| | | | **Spoilage** | | | **Colour** | **Creaming** | | **Fat creep** | | **Sediment** | | **Serum** | | **Gelation** | |
|---|---|---|---|---|---|---|---|---|---|---|---|---|---|---|---|---|
| | **Code** | **Age (M)** | **Bloating** | **Curdling** | **Other** | | **Separation** | **post-dispersion** | **Fat creep** | **post-dispersion** | **Quiescent** | **post-dispersion** | **Separation** | **post-dispersion** | **Gelation** | **post-dispersion** |
| *Standard Product* | Room Temperature | 6 | 0 | 0 | - | **light beige /** cream colour | 0 | 0 | 0 | 0 | 0 | 0 | 0 | 0 | 0 | 0 |
| | Accelerrated (37DegC) | 6 | 0 | 0 | - | light beige / cream colour | 0 | 0 | 0 | 0 | 0 | 0 | 0 | 0 | 0 | 0 |
| *Probiotic UHT* | Room Temperature | 6 | 0 | 0 | - | light beige / cream colour | 0 | 0 | 0 | 0 | 0 | 0 | 0 | 0 | 0 | 0 |
| *Protein Milk* | Accelerrated (37DegC) | 6 | 0 | 0 | - | light beige / cream colour | 0 | 0 | 0 | 0 | 0 | 0 | 0 | 0 | 0 | 0 |
| | | | | | | | | | | | | | | | | |

| | | | **Spoilage** | | | **Colour** | **Creaming** | | **Fat creep** | | Sediment | | **Serum** | | **Gelation** | |
|---|---|---|---|---|---|---|---|---|---|---|---|---|---|---|---|---|
| | **Code** | **Age (M 12)** | **Bloating** | **Curdling** | **Other** | | **Separation** | **post-dispersion** | **Fat creep** | **post-dispersion** | **Quiescent** | **post-dispersion** | **Separation** | **post-dispersion** | **Gelation** | **post-dispersion** |
| *Standard Product* | Room Temperature | 12 | 0 | 0 | - | light beige / cream colour | 0 | 0 | 0 | 0 | 0 | 0 | 0 | 0 | 0 | 0 |
| | Accelerrated (37DegC) | | | | | | | | | | | | | | | |
| *Probiotic UHT* | Room Temperature | 12 | 0 | 0 | - | light beige / cream colour | 0 | 0 | 0 | 0 | 0 | 0 | 0 | 0 | 0 | 0 |
| *Protein Milk* | Accelerrated (37DegC) | | | | | | | | | | | | | | | |

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method of producing a composition of dry spray englobed microparticles in a process comprising:
fluidising an active agent, for example live probiotic, and a carrier material on a bed of a fluidised bed dryer to produce agglomerates of sub-microparticles comprising live probiotic and carrier material;
coating the agglomerates of sub-microparticles by bottom-spraying denatured food grade protein on to the agglomerates on the fluidised bed to form spray englobed microparticles; and
drying the spray englobed microparticles on the fluidised bed.

2. A method according to claim 1, in which the coating step comprises a step of top-spraying food grade protein on to the agglomerates prior to the bottom-spraying step.

3. A method according to claim 1 or 2, in which the coating step comprises bottom spraying a solution of a polymerisation agent on to the coating of denatured food grade protein.

4. A method according to claim 3, in which the polymerisation agent is selected from divalent metal salt such as calcium chloride or an acid such as citric acid.

5. A method according to claim 3 or 4, in which the coating step comprises a drying step to reduce the moisture content of the microparticles before the polymerisation agent is bottom sprayed on to the denatured food grade protein coat.

6. A method according to claim 5, in which the drying step comprises reducing the moisture content of the microparticles by 25-35%.

7. A method according to claim 3, in which the polymerisation agent is bottom sprayed on to the coating of denatured food grade protein while the coating is still wet.

8. A method according to any preceding claim, in which the process comprises a plurality of coating steps, in which each step comprises coating the agglomerates with denatured food grade protein, optionally drying the microparticles, and then spraying polymerisation agent on to the coating.

9. A method according to claim 8, in which the process comprises a drying step to reduce the moisture content of the microparticles in between each coating step.

10. A method according to any preceding claim, in which the fluidisation step comprises fluidising live probiotic, a carrier material and a binder on a bed of a fluidised bed dryer, in which the binder is selected from lipid and denatured protein.

11. A method according to any preceding claim, in which the agglomerates of sub-microparticles are produced in a top-spraying fluidising process.

12. A method according to any preceding claim, in which the fluidising step comprises adding carrier material to the bed of the fluidised bed, fluidising the carrier material, adding live probiotic to the bed of the fluidised bed, and fluidising the carrier material and live probiotic on the bed to form sub-microparticles, and adding a binder to the bed of the fluidised bed, and fluidising the binder and sub-microparticles to form agglomerates.

13. A method according to claim 10 or 12, in which the binder is sprayed on to the fluidised bed.

14. A method according to any preceding claim, in which the drying step comprises drying the spray englobed microparticles to a water activity (Aw) of less than 0.2.

15. A method according to claim 10, 12 or 13, in which the binder comprises denatured food grade protein, and the method comprises a step of top-spraying a polymerising salt onto the agglomerates prior to the coating step.
